# EUROPEAN PATENT SPECIFICATION

(11) **EP 0 729 448 B1**
(45) Date of publication and mention of the grant of the patent: **14.10.1998**
(21) Application number: 95901407.7
(22) Date of filing: 17.11.1994
(51) Int. Cl.: C07C 67/04, C07C 69/007, C07C 69/54

(54) **PROCESS FOR THE PREPARATION OF ETHYLENICALLY UNSATURATED COMPOUNDS**
VERFAHREN ZUM HERSTELLEN VON ETHYLENISCH UNGESÄTTIGTEN VERBINDUNGEN
PROCEDE DE PREPARATION DE COMPOSES NON SATURES D'ETHYLENE

(30) Priority: 17.11.1993 EP 93203220
(43) Date of publication of application: 04.09.1996
(73) Proprietor: SHELL INTERNATIONALE RESEARCH MAATSCHAPPIJ B.V., 2596 HR Den Haag (NL)
(72) Inventor: DRENT, Eit, NL-1031 CM Amsterdam (NL); JAGER, Willem, Wabe, NL-1031 CM Amsterdam (NL)
(86) International application number: EP9403850
(87) International publication number: WO9514001

(56) References cited:
- EP-A- 0 512 656
- EP-A- 0 622 352
- JOURNAL OF ORGANIC CHEMISTRY., vol.52, no.11, 29 May 1987, EASTON US pages 2230 - 2239 TAKE-AKI MUTSIDO ET AL. 'RUTHENIUM-CATALYZED SELECTIVE ADDITION OF CARBOXYLIC ACIDS TO ALKYNES.A NOVEL SYNTHESIS OF ENOL ESTERS' cited in the application

## Description

The invention relates to a process for the preparation of ethylenically unsaturated compounds by reaction of an acetylenically unsaturated compound and/or an 1,2-alkadiene with a Bronsted acid in the presence of a ruthenium containing catalyst.

The ruthenium-catalyzed addition of a Bronsted acid to the unsaturated bond of an acetylenically unsaturated compound is known.

In Organometallics (1983), 2, pp. 1689-1691, by M. Rotem and Y. Shvo, the addition of carboxylic acids to alkynes, in particular internal alkynes such as diphenylacetylene, is disclosed. With the applied ruthenium-complexes, viz., [Ru₃(CO)₁₂] and [Ru(CO)₂(CH₃CO₂)]ₙ, the reaction proceeds at low rate, whereby reaction times as long as 17 to 22 hours are recorded. According to these authors, a more simple catalyst such as RuCl₃ is totally inactive.

On the other hand, it is disclosed in Tetrahedron letters, Vol. 27, No. 52, pp. 6323-6324 (1986), by Chr. Ruppin and P.H. Dixneuf, that saturated and unsaturated carboxylic acids can be added to terminal alkynes, catalyzed by RuCl₃.3H₂O and RuCl₂(PMe₃)(p-cymene). However, here again long reaction times (15 hours) were required and the reaction rate was low.

The use of ruthenium(II)-containing catalysts, viz. bis(η⁵-cyclooctadienyl)ruthenium(II) (trialkylphosphine) (maleic anhydride), is disclosed by T. Mitsudo et al. in J. Org. Chem. (1987), 52, pp. 2230-2239. For the addition of unsaturated carboxylic acids, the presence of maleic acid in the catalyst system is not essential, but in the absence of maleic acid no reaction was observed with saturated carboxylic acids. Whereas the recorded reaction times are shorter (4 hours), the yields obtained show, that the reaction proceeds at relatively low catalyst turnover rate.

According to EP-A-0,512,656 a Bronsted acid may advantageously be added to an acetylenically unsaturated compound with the aid of a heterogeneous catalyst comprising ruthenium, preferably in the zero-valent state, supported on an inert porous support. The working examples are carried out with a ruthenium on carbon catalyst and with ethyne as the acetylenically unsaturated compound.

It has now been found that very attractive results, in particular as regards the reaction rate, are obtained by using a catalyst based on a combination of zero-valent ruthenium and a ligand containing trivalent phosphorus. Moreover, this combination exhibits good catalytic activity for the addition of a Bronsted acid to an allenically unsaturated bond.

The invention may be defined as relating to a process for the preparation of ethylenically unsaturated compounds by contacting an acetylenically unsaturated compound and/or an 1,2-alkadiene with a Bronsted acid in the presence of a catalyst based on zero-valent ruthenium and a trivalent phosphorus atom containing compound acting as a bidentate ligand and having an organic bridging group containing 1 atom in the bridge between the phosphorus atom and the co-ligating atom.

The acetylenically unsaturated compounds which are suitable as starting material in the process of the invention include internal and terminal alkynes which may be unsubstituted or substituted with one or more substituents that do not interfere with the reaction with the Bronsted acid. The alkynes typically have from 2 to 10 carbon atoms. Examples of suitable substituents are alkoxy groups, typically with 1 to 4 carbon atoms, aryloxy groups and halogen atoms.

Suitable internal acetylenes include butyne-2, pentyne-2, hexyne-2, hexyne-3, 1-phenylpropyne-1 and diphenyl-acetylene. Preferably a terminal alkyne is used, for example ethyne, propyne, butyne-1 or phenylacetylene. Propyne represents a particularly preferred starting material.

Commercially available propyne feedstocks usually comprise minor amounts of other unsaturated compounds, such as 1,2-propadiene. An advantage of the process of the invention consists in that the catalyst is also active in the reaction of an 1,2-alkadiene with a Bronsted acid. Consequently, if a commercial propyne feed is used that contains both propyne and 1,2-propadiene, the removal of the 1,2-alkadiene prior to the reaction involving the acetylenically unsaturated compound, can be omitted.

In the present specification, a Bronsted acid is defined as a compound which can release one or more protons. Bronsted acids therefore include inorganic acids and organic acids, alcohols, amino compounds of sufficient acidity, hydroxy esters, amides and imides.

Preferred Bronsted acids are carboxylic acids, both saturated and unsaturated carboxylic acids being suitable. Typical examples of suitable carboxylic acids are acetic acid, propionic acid, butyric acid, isobutyric acid, pivalic acid, acrylic acid, methacrylic acid, crotonic acid, benzoic acid, phthalic acid, isophthalic acid and terephthalic acid. Unsaturated carboxylic acids are most preferred. In particular methacrylic acid has proved to be a very attractive starting material.

Examples of other suitable Bronsted acids include alcohols, such as methanol, ethanol, propanol-1 and propanol-2, phenol, hydroquinone and resorcinol, amino compounds, such as primary aliphatic amines and secondary aromatic amines, amides or imides, such as 2-pyrrolidone, succinimide and toluenesulphonamide.

The source of zero-valent ruthenium is not critical. Metallic ruthenium may be used, conveniently in finely divided form and supported on a suitable carrier. If desired, a zero-valent ruthenium compound may be used, obtained by reduction of a ruthenium(II), or a ruthenium(III)-compound. Preferably, a zero-valent ruthenium complex is used, such as complexes of ruthenium with cyano, carbon monoxide or ammonia groups. In particular preferred are complexes containing zero-valent ruthenium and carbon monoxide, such as [Ru(CO)₅] and [Ru₃(CO)₁₂].

The trivalent phosphorus compound participating in the catalysts of the invention, may be any phosphorus compound having a free electron pair at the phosphorus atom enabling the phosphorus compound to act as a ligand by complexing with ruthenium. Thus, suitable phosphorus compounds include phosphites and phosphines.

Preferably, such compounds are represented by the general formula

R¹R²P-R-M- R³R⁴ (I)

wherein M represents a phosphorus or nitrogen atom, each of R¹, R², R³ and R⁴ independently represents a substituted or non-substituted hydrocarbyl group and R represents an organic bridging group containing 1 atom in the bridge.

Preferably, M represents a phosphorus atom, in which case the compounds of formula (I) are bisphosphines.

Each of R¹, R², R³ and R⁴ may represent a substituted or non-substituted aryl, alkaryl, aralkyl, alkyl, or cycloalkyl group. Also, R¹ together with R² and/or R³ together with R⁴ may form cyclic structures that are linked to the P and/or M atom. Each aryl, alkaryl or aralkyl group represented by R¹, R², R³ or R⁴, typically has from 6 to 14, in particular from 6 to 10 carbon atoms. Aliphatic groups, represented by one or more of R¹, R², R³ and R⁴, usually comprise from 1 to 8, in particular from 1 to 4 carbon atoms.

Substituents may be conveniently selected from halogen atoms, cyano groups, alkoxy groups containing from 1 to 4 carbon atoms and hydroxy groups.

In the compounds of formula (I) the bridging group, represented by R, preferably consists of a single carbon atom. Typically the bridging group is a methylene group, but if one or both hydrogen atoms are replaced by substituents, R may also be a methanetriyl or methanetetrayl group.

Examples of recommended compounds of formula (I) are bis(dialkylphosphino)alkanes, such as bis(di-n-butylphosphino)-methane and bis(di-sec.butylphosphino)methane; bis(diarylphosphino)-alkanes, such as bis(diphenylphosphino)methane and bis(dinaphthylphosphino)methane; dialkylamino-diarylphosphinoalkanes, such as dimethylamino-diphenylphosphinomethane and diethylamino-diphenylphosphinomethane; and bis(cycloalkylenephosphino)alkanes, such as bis(cyclooctylenephosphino)methane and bis(cyclohexylenephosphino)methane.

A special category of compounds acting as bidentate ligands is formed by compounds wherein the phosphorus atom is linked to a carbon atom of a nitrogen-containing aromatic group, for example a pyridyl group, pyridazinyl group or a pyrimidinyl group. The phosphorus atom may be linked to more than one or these groups, if so desired, or to one nitrogen-containing aromatic group and two other groups, such as phenyl groups. Examples of preferred ligands of this category are 2-diphenylphosphinopyridine, 2-diphenylphosphino-6-methylpyridine, 2-dimethylphosphinopyridine and 2-diphenylphosphinoquinoline.

The amount of trivalent phosphorus containing compound is not critical. However, it is recommended to apply it in an amount of 0.5 to 20, preferably 1 to 5 moles per mole atom of ruthenium.

The catalyst system used in the process of the invention, may be homogeneous or heterogeneous. If a heterogeneous catalyst is used, the zero-valent ruthenium and the trivalent phosphorus compound are supported on an inert carrier. Suitable carrier materials include refractory oxides, such as alumina, silica, titania and silica-alumina and carbon. If supported, then supports, substantially consisting of carbon, are preferred.

The Bronsted acid may usually constitute the reaction medium, so that no additional solvent or diluent is required. If desired, an additional diluent, such as aprotic liquids, may be used. Suitable examples are ethers, such as diethylether and 2,5,8-trioxanonane (diglyme) and ketones, such as methylethylketone and methylisobutylketone.

The addition reaction is conveniently carried out at elevated temperatures, usually in the range of 50 - 200 °C. Preferred reaction temperatures are in the range of 80 - 160 °C. When acetylenically unsaturated compounds are used that are in the vapour state at the prevailing reaction temperature, the pressure is conveniently maintained in the range of 1 to 50 bar absolute. Usually, the partial pressure of the acetylenically unsaturated compound can be maintained below 10 bar, but for safety reasons, inert gases, such as nitrogen are often added, bringing the total pressure up to a value of say 25 bar.

The products obtained by addition of a Bronsted acid to an unsaturated bond of the starting material may be linear or branched, depending, inter alia, on the selected catalyst combination. In particular, the non-linear addition products are of interest as intermediates in the preparation of speciality chemicals and as monomers in free radical initiated polymerization processes. It is believed that the selectivity with respect to a linear or non-linear addition product is affected by steric, as well as electronic factors involved in the coordination between the zero valent ruthenium and the specific phosphorus ligand. Thus, it has been observed that the use of a compound comprising a pyridyl group results in a high selectivity with respect to non-linear addition products. It will of course be appreciated that with acetylene as starting material, only linear addition products will be formed.

The invention will be illustrated by the following non-limiting examples.

### Example 1 (for comparison, not according to the invention)

After being flushed, a 250 mL autoclave was charged with 30 mL of methacrylic acid, 50 mL of 2,5,8-trioxanonane, 2 bar of propyne, 106 mg of a zero valent ruthenium-carbon monoxide complex [Ru₃(CO)₁₂] and 260 mg of triphenylphosphine. For safety reasons 20 bar of nitrogen was added. Upon sealing, the contents of the autoclave were heated to 130 °C for 5 hours. Subsequently the autoclave was cooled to ambient temperature and the contents were analyzed by means of gas-liquid chromatography. It was found that 35% of the propyne had been converted and that the selectivity to isopropenylacrylate was 91%.

The reaction rate was calculated to be 42 mol product per mole atom of ruthenium and per hour.

The catalyst composition, reaction time, selectivity to the isoalkenylester and reaction rate are shown in Table 1.

### Examples 2-15

A number of (comparative) experiments were carried out, substantially as described in Example 1, but using other phosphorus compounds, instead of triphenylphosphine. The catalyst composition, reaction time, selectivity to the isoalkenylester and reaction rate are shown in Table 1.

### Example A (for comparison, not according to the invention)

An experiment was carried out, substantially as described in Example 1, with the difference that instead of triphenylphosphine, bis(dimethylamino)ethane was used as catalyst component.

The catalyst composition, reaction time, selectivity to the isoalkenylester and reaction rate are shown in Table 1.

From the results it can be seen that by using a catalyst ligand which does not contain trivalent phosphorus, the selectivity to the isoalkenylester was only 54% and that the reaction proceeded at a very low rate. In addition, catalysts based on bidentate ligands having 1 bridging atom clearly outperformed catalysts not according to the invention.

### Examples 16-19

A number of experiments were carried out, substantially as described in Example 12, but using another Bronsted acid, instead of methacrylic acid and (as regards Examples 18 and 19) another unsaturated starting material, instead of propyne.

The reactants, reaction time, selectivity to the isoalkenyl ester and reaction rate are shown in Table 2.

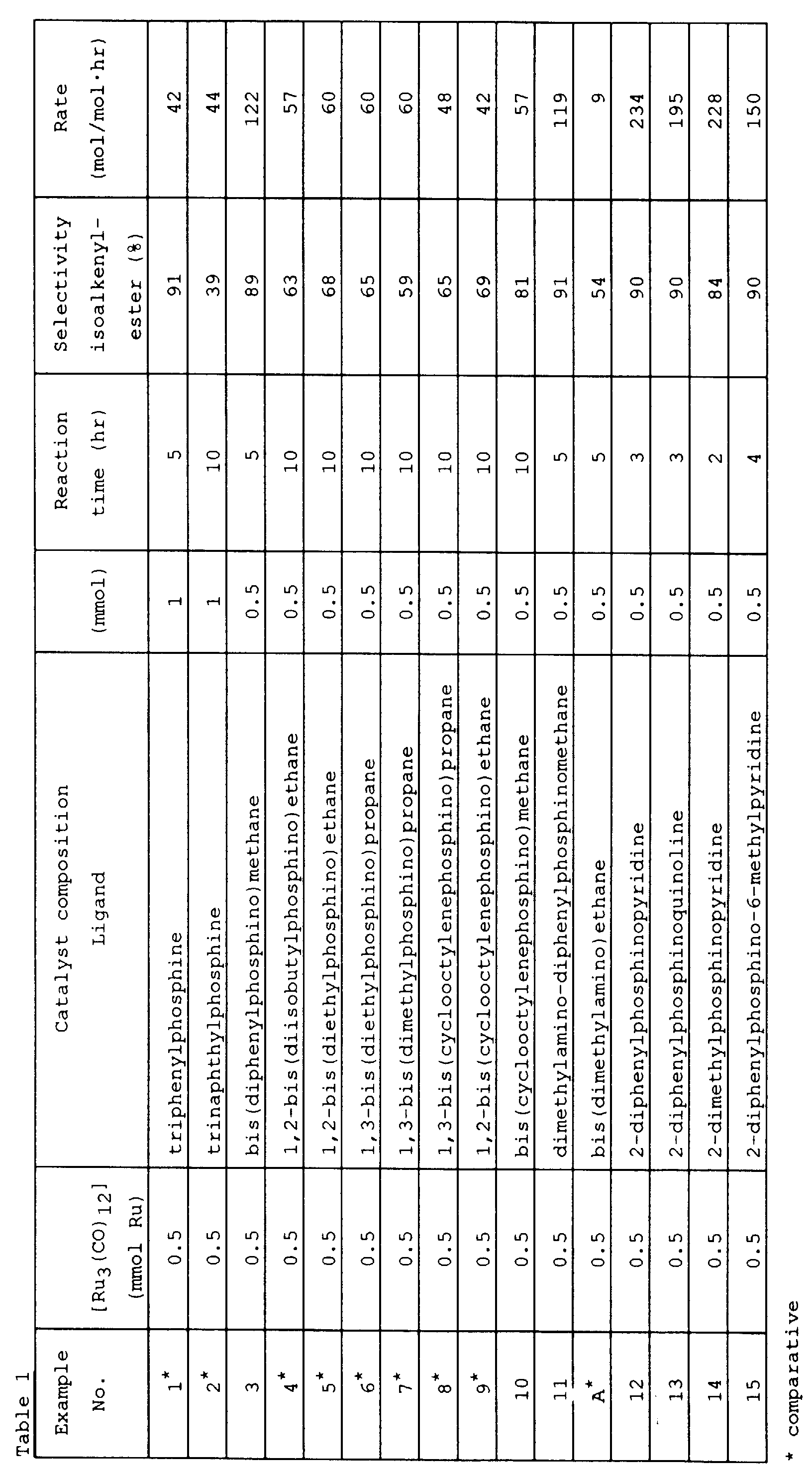

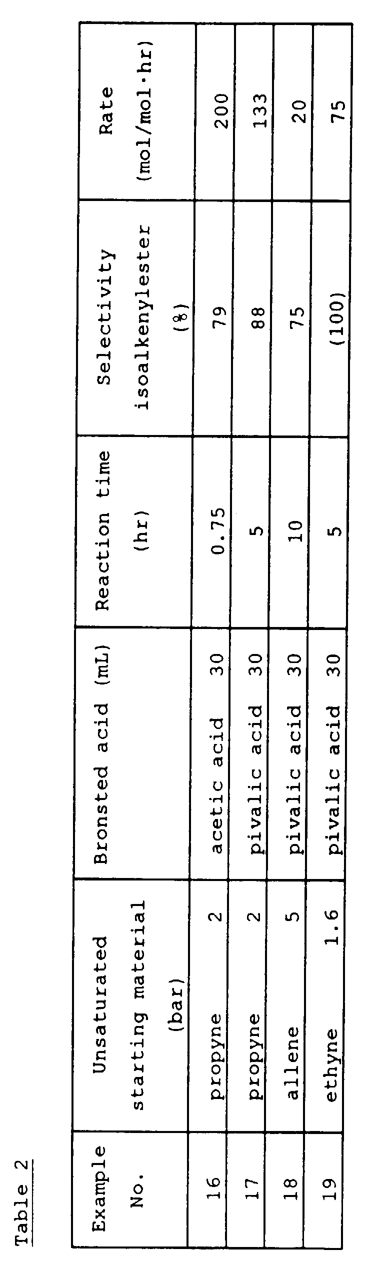

## Claims

1. A process for the preparation of ethylenically unsaturated compounds by contacting an acetylenically unsaturated compound and/or an 1,2-alkadiene with a Bronsted acid in the presence of a catalyst based on zero-valent ruthenium and a trivalent phosphorus atom containing compound acting as a bidentate ligand and having an organic bridging group containing 1 atom in the bridge between the phosphorus atom and the co-ligating atom.

2. A process as claimed in claim 1, wherein propyne is used.

3. Process as claimed in claim 1 or 2, wherein as Bronsted acid a carboxylic acid, preferably an unsaturated carboxylic acid is used.

4. A process as claimed in claim 3, wherein methacrylic acid is used.

5. A process as claimed in any one of claims 1 to 4, wherein as catalyst a complex is used, containing zero-valent ruthenium, carbon monoxide and the trivalent phosphorus containing compound acting as bidentate ligand.

6. A process as claimed in any of claims 1 to 5, wherein the trivalent phosphorus containing compound is a phosphine.

7. A process as claimed in any of claims 1 to 6, wherein as bidentate ligand a compound is used having the general formula
R¹R²P-R-M-R³R⁴ (I)
wherein M represents a phosphorus or nitrogen atom, each of R¹, R², R³ and R⁴ independently represents a substituted or non-substituted hydrocarbyl group or R¹ together with R² and/or R³ together with R⁴ form cyclic structures that are linked to the P and/or M atom and R represents an organic bridging group containing 1 atom in the bridge.

8. A process as claimed in claim 7, wherein in the compound of formula (I), M represents a phosphorus atom.

9. A process as claimed in any of claims 1 to 6, wherein in the compound the phosphorus atom is linked to a 2-pyridyl group.

10. A process as claimed in claim 9, wherein the compound is 2-diphenylphosphinopyridine or 2-dimethylphosphinopyridine.

## Patentansprüche

1. Verfahren zur Herstellung von ethylenisch ungesättigten Verbindungen durch Inkontaktbringen einer acetylenisch ungesättigten Verbindung und/oder eines 1,2-Alkadiens mit einer Brönstedsäure in Anwesenheit eines Katalysators auf der Basis von nullwertigem Ruthenium und einer ein dreiwertiges Phosphoratom enthaltenden Verbindung, die als ein Bidentatligand wirkt und eine organische Brückengruppe aufweist, die ein Atom in der Brücke zwischen dem Phosphoratom und dem co-ligierenden Atom enthält.

2. Verfahren nach Anspruch 1, worin Propin zum Einsatz gelangt.

3. Verfahren nach Anspruch 1 oder 2, worin eine Carbonsäure, vorzugsweise eine ungesättigte Carbonsäure als Brönstedsäure verwendet wird.

4. Verfahren nach Anspruch 3, worin Methacrylsäure verwendet wird.

5. Verfahren nach einem der Ansprüche 1 bis 4, worin als Katalysator ein Komplex verwendet wird, der nullwertiges Ruthenium, Kohlenmonoxid und die als Bidentatligand wirkende Verbindung mit einem Gehalt an dem dreiwertigen Phosphor enthält.

6. Verfahren nach einem der Ansprüche 1 bis 5, worin die den dreiwertigen Phosphor enthaltende Verbindung ein Phosphin ist.

7. Verfahren nach einem der Ansprüche 1 bis 6, worin als Bidentatligand eine Verbindung mit der allgemeinen Formel
R¹R²P-R-M-R³R⁴ (I)
verwendet wird, worin M ein Phosphor- oder Stickstoffatom darstellt, jeder der Reste R¹, R², R³ und R⁴ unabhängig eine substituierte oder unsubstituierte Hydrocarbylgruppe bedeutet oder R¹ zusammen mit R² und/oder R³ zusammen mit R⁴ zyklische Strukturen ausbilden, die an das P- und/oder M-Atom gebunden sind und R eine organische Brückengruppe bedeutet, die ein Atom in der Brücke enthält.

8. Verfahren nach Anspruch 7, worin in der Verbindung der Formel (I) M für ein Phosphoratom steht.

9. Verfahren nach einem der Ansprüche 1 bis 6, worin in der Verbindung das Phosphoratom an eine 2-Pyridylgruppe gebunden ist.

10. Verfahren nach Anspruch 9, worin die Verbindung 2-Diphenylphosphinopyridin oder 2-Dimethylphosphinopyridin ist.

## Revendications

1. Procédé de préparation de composés éthyléniquement insaturés par la mise en contact d'un composé acétyléniquement insaturé et/ou d'un 1,2-alcadiène avec un acide de Bronsted en présence d'un catalyseur à base de ruthénium zéro valant et d'un composé contenant un atome de phosphore trivalent, servant de ligand bidentate possédant un groupe de pontage contenant un atome dans le pont entre l'atome de phosphore et l'atome co-liant.

2. Procédé suivant la revendication 1, caractérisé en ce que l'on utilise du propyne.

3. Procédé suivant la revendication 1 ou 2, caractérisé en ce que l'on utilise, à titre d'acide de Bronsted, un acide carboxylique, de préférence un acide carboxylique insaturé.

4. Procédé suivant la revendication 3, caractérisé en ce que l'on utilise l'acide méthacrylique.

5. Procédé suivant l'une quelconque des revendications 1 à 4, caractérisé en ce que l'on utilise, à titre de catalyseur, un complexe contenant du ruthénium zéro valant, du monoxyde de carbone et le composé contenant du phosphore trivalent agissant à titre de ligand bidentate.

6. Procédé suivant l'une quelconque des revendications 1 à 5, caractérisé en ce que le composé contenant du phosphore trivalent est une phosphine.

7. Procédé suivant l'une quelconque des revendications 1 à 6, caractérisé en ce qu'on utilise, à titre de ligand bidentate, un composé répondant à la formule générale :
R¹R²P-R-M-R³-R⁴ (I)
dans laquelle M représente un atome de phosphore ou d'azote, chacun des symboles R¹, R², R³ et R⁴ représente indépendamment un radical hydrocarbyle substitué ou non substitué ou bien R¹ ensemble avec R² et/ou R³ ensemble avec R⁴ forment des structures cycliques qui sont liées à l'atome P et/ou M et R représente un groupe de pontage organique contenant un atome dans le pont.

8. Procédé suivant la revendication 7, caractérisé en ce que dans le composé de la formule (I) M représente un atome de phosphore.

9. Procédé suivant l'une quelconque des revendications 1 à 6, caractérisé en ce que dans le composé, l'atome de phosphore est lié à un radical 2-pyridyle.

10. Procédé suivant la revendication 9, caractérisé en ce que le composé est la 2-diphénylphosphinopyridine ou la 2-diméthylphosphinopyridine.
